# EUROPEAN PATENT APPLICATION

(11) **EP 4 851 283 A1**
(43) Date of publication of application: **09.12.2026**
(21) Application number: 25180684.0
(22) Date of filing: 04.06.2025
(51) Int. Cl.: C12N 11/14, C12N 9/16, C12N 9/22, C12N 15/86

(54) **BIOPHARMACEUTICAL PRODUCTION PROCESS APPLYING A FUNCTIONALIZED FILTER MATERIAL**

(71) Applicant: Sartorius Stedim Biotech GmbH, 37079 Göttingen (DE)
(72) Inventor: Quaas, Bastian, 37079 Göttingen (DE); Austerjost, Jonas, 37079 Göttingen (DE)
(74) Representative: Müller-Boré & Partner Patentanwälte PartG mbB

(57) **Abstract**

The present invention relates to a biopharmaceutical production process applying a functionalized filter material comprising a filter material and one or more hydrolases covalently bonded to the filter material.

## Description

The present invention relates to a biopharmaceutical production process applying a functionalized filter material comprising a filter material and one or more hydrolases covalently bonded to the filter material.

Biopharmaceutical production is inherently complex, deploying engineered cells to generate therapeutic agents, with the concurrent introduction of undesirable impurities during the cultivation and downstream processing stages, requiring sophisticated and multifaceted purification strategies. Particularly, as the industry evolves to encompass newer modalities such as viral vaccines and gene therapies, the implementation of innovative technologies becomes imperative.

One predominant and persistent challenge within bioprocessing is the clogging of filters due to the accumulation of cells, cellular debris, and other particulate matter, which complicates and hampers the purification stages of biopharmaceutical production. This clogging leads to reduced filtration efficiency, necessitates frequent filter replacements, and increases both operational downtime and costs. Furthermore, failure to adequately remove such impurities compromises the quality, safety, and efficacy of the final biopharmaceutical products, emphasizing the need for more innovative and effective solutions in filtration and purification.

Filter aids (FAs), such as Diatomaceous Earth (DE), which is renowned for its unique microporous structure, have proven to be an effective solution for mitigating filtration challenges. When integrated directly into the cell culture broth, it acts as an adsorptive medium, trapping cells and cell debris, thus preventing e.g. filter clogging and enhancing the reliability and efficiency of downstream operations. However, the adsorptive character of FAs is restricted.

Purification stages can also include further devices such as incubation chambers. In such chambers, hydrolases can be used for cleaving host cell impurities like proteins and DNA/RNA. However, the use of such enzymes results in further separate purification steps for separating the enzymes. Moreover, a large amount of hydrolases is needed, since hydrolases are commonly not reused after separation.

Thus, there is a need to further advance purification stages of biopharmaceutical production processes.

Thus, the technical problem underlying the present invention is to provide means for improving the efficiency in biopharmaceutical production processes, in particular in downstream processing thereof.

The solution to the above technical problem is achieved by the embodiments characterized in the claims.

In particular, the present invention relates to a biopharmaceutical production process comprising a step of contacting a process liquid with a functionalized filter material, wherein the functionalized filter material comprises a filter material and one or more hydrolases covalently bonded to the filter material.

The present invention is able to combine adsorptive filtration capabilities of filter materials with the enzymatic prowess of hydrolases for the degeneration of undesired contaminating biomolecules such as host cell impurities like proteins and DNA/RNA in biopharmaceutical production processes. This preferably increases the overall purification effectiveness of the functionalized filter material significantly in such processes, e.g. during filtration by e.g. avoiding premature filter blockage due to clogging. Moreover, the integration of adsorption and enzymatic degradation in one step and entity reduces the complexity and resource intensiveness of purification processes.

The enhanced selectivity and specificity enabled by this approach preferably ensure targeted impurity degradation, preserving the structural and functional integrity of the desired bioproducts and augmenting overall product purity and yield. Additionally, this methodology preferably integrates adsorptive and enzymatic degradation processes, optimizing and streamlining operational workflows, reducing process times, and alleviating the complexities associated with downstream processes.

Moreover, the above approach allows significant operational cost and resource efficiency by reducing the necessity for extensive purification steps and frequent filter replacements. Its adaptability and scalability make it an ideal solution for various biopharmaceutical modalities, with the incorporation of suitable hydrolases. The resultant improvement in product quality, by ensuring the efficient and precise removal of host cell impurities and of the functionalized filter material after application, aligns with the stringent regulatory standards, ensuring the production of safe and high-quality biopharmaceuticals. The use of hydrolase-functionalized filter materials eliminates the need for separate purification steps for the enzymes when compared to processes using free hydrolases. Moreover, less hydrolase is needed, since the hydrolase is retained in the respective unit, such as incubation chambers, on the hydrolase-functionalized filter materials. In contrast, to the use of e.g. microbeads, the application of porous functionalized filter material, such as functionalized diatomaceous earth, can be particularly advantageous as blocking of e.g. incubation chambers can be avoided due to a high porosity of the functionalized filter material.

Furthermore, the enhanced reliability afforded by this approach preferably minimizes the risk of batch failures and ensures consistency and continuity in biopharmaceutical production cycles. The above approach offers a solution, addressing the intertwined challenges of e.g. filtration and purification in biopharmaceutical production. It preferably allows elevating bioprocessing standards and enhanced efficiency, reliability, and purity in the delivery of high-quality biopharmaceutical products.

The term "process liquid" is not particularly limited and encompasses all kind of process liquids in biopharmaceutical production processes. Preferably, the process liquid is a liquid in downstream processing. For example, the process liquid may be cell lysates, cell broth, cell broth supernatant, and pre-purified liquid (e.g. via TFF, chromatography). Examples of components included in the process liquid apart from biopharmaceutical products are cell debris, host cell proteins, host cell DNA, and host cell RNA. The functionalized filter material preferably performs adsorptive and enzymatic degradation of components other than the biopharmaceutical product of interest in the process liquid.

The term "biopharmaceutical production process" is not particularly limited and encompasses all kind of biopharmaceutical production processes. Examples of biopharmaceutical products obtained by the biopharmaceutical production process are therapeutic proteins, such as mAbs, viral vectors, such as AAV, viruses, such as lentivirus, nucleic acid-based products, such as mRNA, pDNA, olignucleotides, and siRNA, exosomes, such as extracellular vesicles, and lipid-nanoparticles, such as RNA vaccines and liposomes. Preferably, the biopharmaceutical product obtained by the biopharmaceutical production process is selected from AAV, mABs and mRNA. For example, the biopharmaceutical production process may be a process involving the cultivation and growth of (host) cell cultures, which may produce the target product (biopharmaceutical product) of interest.

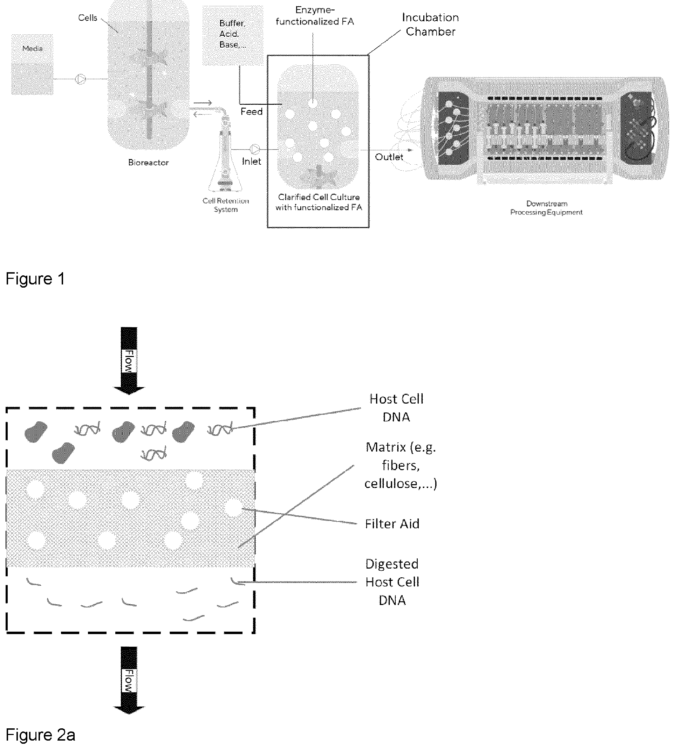

An example for a continuous USP/DSP (upstream processing/downstream processing) process including a cell culture tank with enzyme-functionalized filter materials is depicted in Figure 1. The depicted process illustrates perfusion cultivation in a bioreactor, utilizing a cell retention device, preferably based on an alternating tangential flow (ATF) or TFF system. The clarified cell culture is transferred via a pump to an intermediate tank containing functionalized filter materials, such as functionalized diatomaceous earth (DE) spheres. Depending on the enzymes functionalized on the filter material, specific digestion or hydrolysis reactions occur, effectively reducing impurities such as host cell proteins (HCP), host cell DNA and/or host cell RNA. The stirred tank is directly connected to a multi-column chromatography system, equipped with columns designed for affinity chromatography. The flow from the intermediate tank to the chromatography system can be regulated based on the mass within the tank to ensure optimal process control.

Any hydrolase known in the art can be part of the functionalized filter material. For example, the hydrolase of the functionalized filter material may be selected from the group consisting of an esterase, such as a carboxylesterase and a cholinesterase, a lipase, a phospholipase, a peptidase, such as a serine protease, a cysteine protease, an aspartic protease, and a metalloprotease, a nuclease, such as a DNase and a RNase, a glycoside hydrolase, such as an amylase, a cellulase, a lactase, and a pectinase, a phosphatase, such as an alkaline phosphatase and an acid phosphatase, a sulfatase, such as an arylsulfatase, an amidase, such as an urease and an asparaginase, and an epoxide hydrolase, such as a microsomal epoxide hydrolase, as well as combinations of two or more thereof. In a preferred embodiment, the hydrolase is selected from the group consisting of a nuclease and a peptidase, as well as combinations thereof. Preferably, one type of hydrolase is bonded to the filter material. Most preferably, the hydrolase of the functionalized filter material is a nuclease.

Any filter material known in the art can be part of the functionalized filter material. For example, the filter material of the functionalized filter material may be selected from the group consisting of diatomaceous earth, perlite, cellulose fibers, activated carbon, silica gel, glass microfibers, polymeric resins, minerals, such as clays, pumice, and zeolites, preferably diatomaceous earth, perlite, cellulose fibers, and zeolites. In a preferred embodiment, the filter material is selected from the group consisting of a diatomaceous earth and cellulose fibers. Most preferably, the filter material of the functionalized filter material is diatomaceous earth.

The filter material of the functionalized filter material may have a permeability of from 20 mD to 5000 mD. The permeability of the filter material is preferably from 50 mD to 1000 mD, more preferably from 100 mD to 800 mD, most preferably from 200 mD to 500 mD. The permeability can be determined by the method as described in Makarov I.S. et al., Structure, Morphology, and Permeability of Cellulose Films, Membranes, 12, 2022, 297.

The filter material of the functionalized filter material can have different (perforated) three-dimensional shapes, such as spherical, discs, frustules, or other complex structures, such as those resembling ladders, feathers, or needles. The filter material may have a nonuniform particle size distribution. The median value D50, based on volume distribution (e.g., measured by laser diffraction according to ISO 13320, e.g. for particles of irregular shapes), of the particle size distribution of the filter material may be from 0.2 to 2000 µm. For particles with varying dimensions (e.g., rods or fibrous structures), the equivalent circular diameter is determined (by the same method) to standardize measurement. The median value D50 of the particle size distribution of the filter material is preferably from 10 to 2000 µm, more preferably from 100 to 1000 µm, and most preferably from 400 to 700 µm.

According to the present invention, the functionalized filter material comprises a filter material and one or more hydrolases covalently bonded to the filter material. In the functionalized filter material, the filter material is functionalized by the presence of the one or more hydrolases covalently bonded to the filter material. The hydrolase(s) may (each) optionally be covalently bonded to the filter material via a linking group. Such linking group may for example be derived from one or more selected from the group consisting of a 3-aminopropyltriethoxysilane (APTES) and glutaraldehyde. Hydrolases can be covalently immobilized using amino, carboxyl, hydroxyl, thiol, aldehyde, and imidazole groups on the enzyme. The covalent bonding may for example be achieved by the method as described in Novick, S.J. et al., Immobilization of Enzymes by Covalent Attachment, in: Barredo, J.L. (eds) Microbial Enzymes and Biotransformations. Methods in Biotechnology, vol 17, Humana Press.

The number of hydrolases covalently bonded to a filter material in the functionalized filter material is not particularly limited and may for example be from 0.5 g to 5.0 g of enzyme per square centimeter of filter material surface (total surface including pore surface).

The amount of functionalized filter material applied in the biopharmaceutical production process is not particularly limited. Moreover, in a contacting step different types of functionalized filter material may also be applied.

According to the present invention, the biopharmaceutical production process comprises a step of contacting the process liquid with the functionalized filter material. The contacting step can for example be carried out by providing the process liquid to a process unit, such as a (stirred) vessel, (stirred) tank, filter medium, and filter composite, comprising the functionalized filter material. The process liquid is for example processed in flow-through mode (semi) continuously to the next process unit in the process) or in a batch-wise manner, preferably in flow-through mode. In case of a batch-wise manner, the duration is not particularly limited and may for example be from 30 min to 300 min, preferably from 40 min to 70 min. The temperature may for example be from 25°C to 40°C, preferably from 30°C to 37°C. A concentration of Mg²⁺ may for example be from 0.5 mM to 5.0 mM, preferably from 1.0 mM to 2.0 mM. The pH may for example be from 5 to 10, preferably from 7 to 8. In addition to the contacting step (first contacting step (not necessarily timewise/process-order-wise)), the biopharmaceutical production process of the present invention may further comprise one or more additional contacting step(s) before or after the (first) contacting step, wherein the functionalized filter material(s) applied in the additional contacting step(s) are independently the same or different than the functionalized filter material of the first contacting step.

The contacting step(s) may be carried out during downstream processing or upstream processing. Preferably, at least the first contacting step is carried out during downstream processing. At least the first contacting step may be carried out during or directly (i.e. with no other steps in-between) before or after a clarification step.

In the clarification step, the process liquid may be subjected to one or more of filtration and centrifugation. Examples of filtration methods are depth filter filtration, normal flow filter filtration, alternating tangential flow (ATF) filtration, tangential flow filtration (TFF), and tangential flow depth filtration (TFDF). The filter medium used for the filtration may be selected from single- or multi-layered fibers selected from the group consisting of glass microfibers, cellulose acetate fibers, polyamide fibers, polyethersulfone (PES) fibers, polytetrafluoroethylene (PTFE) fibers, polyvinylidenefluoride (PVDF) fibers, polypropylene (PP) fibers, and polysulfone (PSU) fibers. The functionalized filter material may be embedded in the filter medium, e.g. in case a contacting step is carried out during a clarification step.

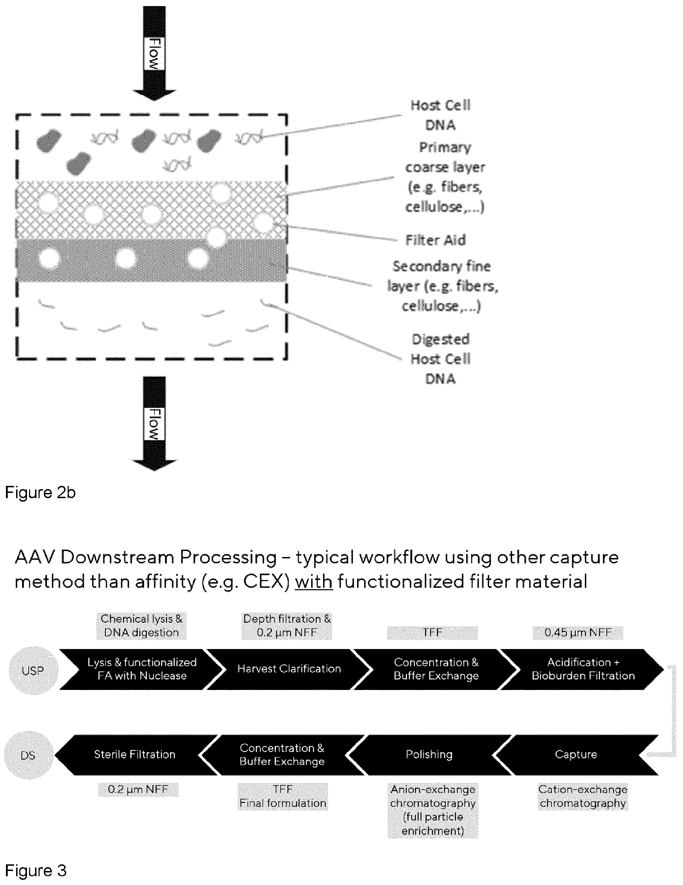

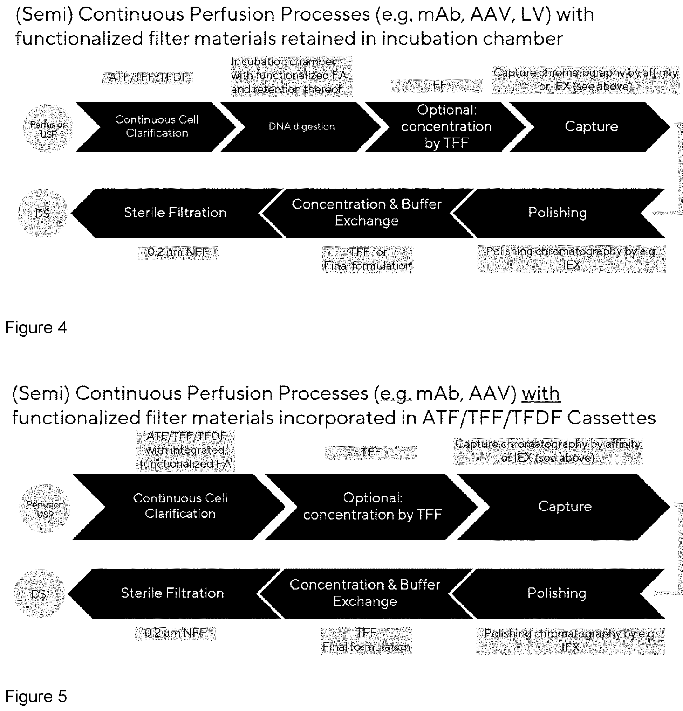

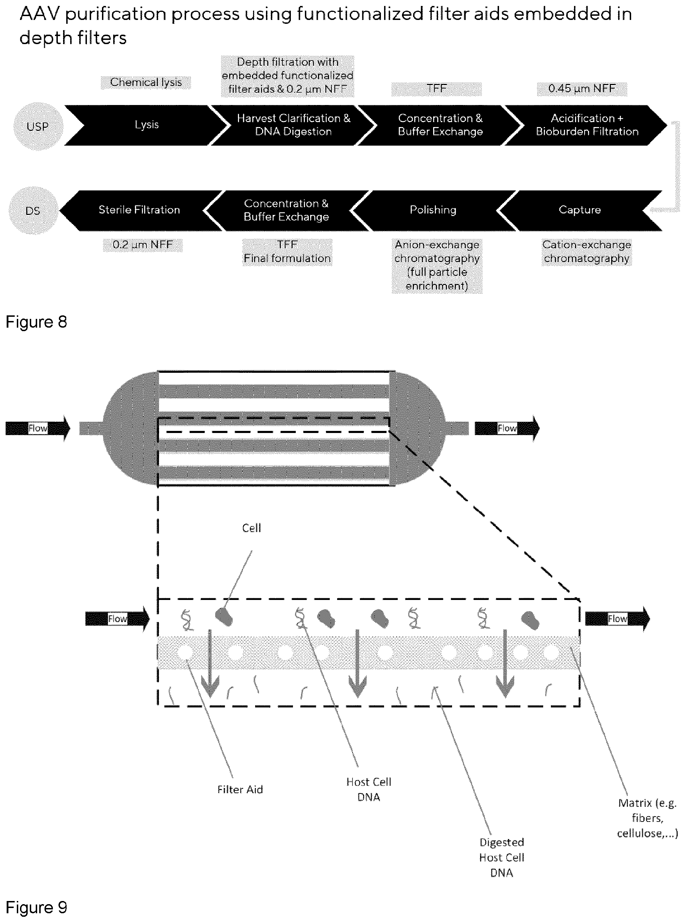

In case a contacting step is carried out during a clarification step, a filter composite may for example also be applied, which comprises a housing and a matrix, wherein the functionalized filter material is embedded in the filter medium, and optionally further comprises a membrane. The matrix may be a single layer or a multi-layer. In case of a double-layered matrix, the upstream layer may be a coarse layer and the downstream layer may be fine layer (in comparison to each other). For example, the coarse layer may have a pore size from 0.5 µm to 20 µm and the fine layer may have a pore size from 0.1 µm to 5.0 µm. Preferred materials include cellulose fibers or reinforced glass fibers for one or all layers of a single layer or a multi-layer matrix. Preferably, in the case of carrying out the contacting step during a clarification step, the filter medium is multi-layered cellulose fibers. The material of the housing is not particularly limited and may be selected from the group consisting of stainless steel, polypropylene, polyethersulfone, and polycarbonate. The functionalized filter material may be evenly distributed in the matrix. Examples of filter composites are shown in Figures 2a and 2b. Whereas Figures 2a and 2b illustrate examples of dead-end filtration devices, Figure 9 depicts a device designed for continuous processes, specifically as a tangential flow filtration (TFF) or (TFDF) module. This module can be used in the processes highlighted in Figure 5.

In case a contacting step is carried out before or after a clarification step, the functionalized filter material may be included in an incubation chamber which may comprise means for retention of the functionalized filter material, such as a membrane.

The contacting step may be incorporated in a step of digesting host cell DNA, host cell RNA, and/or host cell proteins. Host cells are commonly applied for producing target products of interest and after sufficient growth in upstream processing, the cell culture broth can be subjected to downstream processing (batchwise, continuous or semi-continuous) for (finally) obtaining the target product. Apart from cell debris and other particulate matter, host cell DNA or RNA and host cell proteins represent a large fraction of the components included in the process liquid, which can lead to filter clogging etc. in purification processes. Thus, incorporating the contacting step in a step of digesting host cell DNA or RNA and/or host cell proteins preferably allows to significantly improve the overall purification effectiveness. The functionalized filter material preferably comprises nucleases and/or peptidases as hydrolases in this case. The digestion step may be carried out in an incubation chamber, which may comprise means for retention of the functionalized filter material, such as a membrane. The application of porous functionalized filter material, such as functionalized diatomaceous earth, can be particularly advantageous in this case, as these have such a high porosity that blocking of the retention membrane is less likely than by other materials, such as microbeads.

The incubation chamber can be described as a vessel, which may for example be constructed from stainless steel or designed as single-use bags. The incubation chamber can be equipped with a stirrer to ensure the homogenization of the contained liquid and also a temperature control unit. The incubation chamber preferably features an inlet positioned at the bottom and an outlet at the bottom. The outlet is preferably fitted with a membrane to retain the functionalized filter material in the incubation chamber. Additionally, the incubation chamber may include optional feeding lines to introduce other liquids, such as buffers (e.g. also including enyzme substrates/cofactors such as Mg²⁺), acids, or bases, as required (cf. Figure 1).

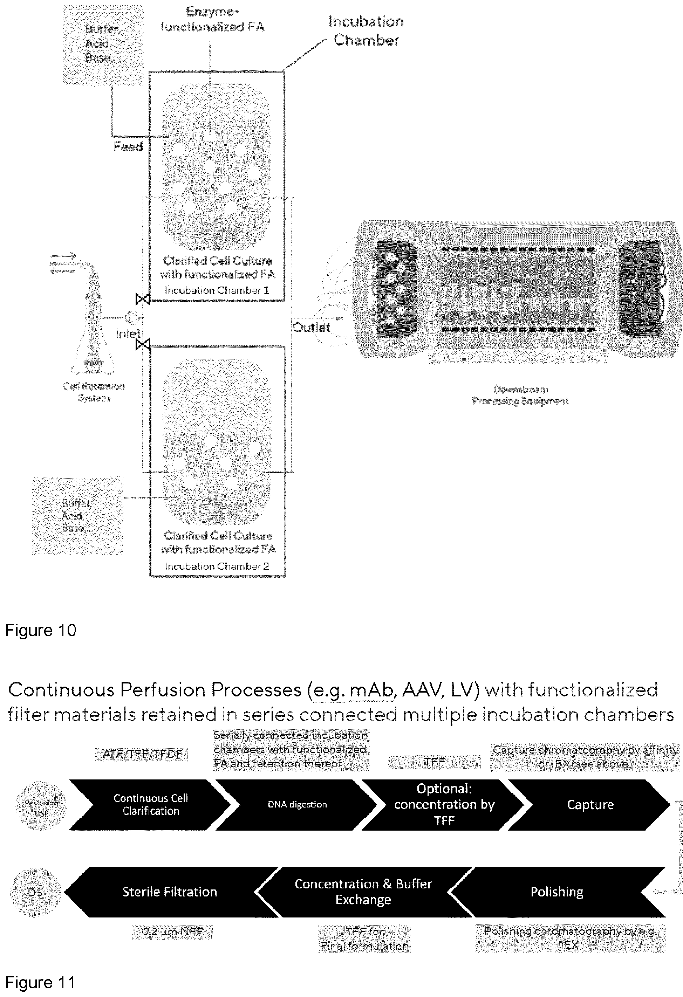

The incubation chamber can be operated in either a continuous or a semi-continuous mode. In the semi-continuous mode, the chamber is filled with process liquid to the targeted liquid level while the outlet remains closed. During or after process loading, the process liquid can be adjusted (e.g. concerning pH) or supplemented with additives such as enzyme cofactors via the feeding line. Once the chamber is fully loaded, the liquid can be retained for a predefined time frame to ensure uniform digestion of host cell DNA/protein by the incorporated functionalized filter material. A digestion time for host cell DNA/protein degradation using functionalized filter material may for example be from 30 min to 90 min, preferably from 45 min to 60 min, in batch mode and may for example be from 15 min to 45 min retention time per chamber in continuous mode. Upon completion of digestion, the processed liquid is discharged through the outlet to the next operational unit, such as a chromatographic unit. The functionalized filter material remains in the chamber, retained by the membrane at the outlet, and can be reused for subsequent cycles. For continuous operation, two or more incubation chambers can be connected in series. Once one chamber is filled and the digestion cycle is initiated, another chamber can be loaded using switching valves at the inlets of the chambers (refer to Figure 10). After digestion in the first chamber is completed, it can be emptied, and digestion in the second chamber can commence, allowing the first chamber to be reloaded with subsequent process liquid (cycling).

A step of lysing host cells may be carried out in the biopharmaceutical production process. Moreover, the lysing step may be carried out before the digestion step, which includes the contacting step, or may be included in the digestion step. Means of lysing host cells are known in the art and can be applied accordingly. Thereby, it is possible to digest DNA, RNA and/or proteins of the lysed host cells and to remove the fragments together with cell debris and larger impurities by the functionalized filter material. Cell debris is preferably separated before by suitable means, such as a depth filter or an ATF module, having no functionalized filter material.

The contacting step may be carried out before one or more chromatography steps, such as capture chromatography (e.g. affinity chromatography), ion exchange (IEX) chromatography, mixed mode chromatography, and reverse phase chromatography. These steps can be applied for further purification for example by binding the target components or removing remaining impurities, ensuring a high purity level. Preferably, the contacting step is carried out before capture chromatography (e.g. affinity chromatography).

The biopharmaceutical production process may comprise further steps, preferably downstream processing steps. Examples of possible further steps are (further) filtration steps, such as TFF and SPTFF.
Fig. 1: Example for a continuous USP/DSP process including an incubation chamber with incorporated enzyme-functionalized filter materials.
Fig. 2a/b: Examples of depth filters including enzyme-functionalized filter materials in a matrix (Fig. 2a: single-layer matrix; Fig. 2b: double-layer matrix).
Fig. 3: AAV Downstream Processing workflow using functionalized filter material.
Fig. 4: (Semi) Continuous Perfusion Processes (e.g. mAb, AAV, LV) with functionalized filter materials retained in incubation chamber.
Fig. 5: (Semi) Continuous Perfusion Processes (e.g. mAb, AAV) with functionalized filter materials incorporated in ATF/TFF/TFDF Cassettes.
Fig. 6: *E. coli* clarification for plasmid purification applying functionalized filter materials.
Fig. 7: mRNA vaccine process with functionalized filter materials.
Fig. 8: AAV purification process in downstream processing using functionalized filter material embedded in depth filters.
Fig. 9: Example of a device designed for continuous processes, specifically as a tangential flow filtration (TFF) or (TFDF) module, including functionalized filter materials in a matrix.
Fig. 10: Example of a device designed for continuous operation, which has two incubation chambers.
Fig. 11: Continuous Perfusion Processes (e.g. mAb, AAV, LV) with functionalized filter materials retained in serially connected incubation chambers.

In the following, there are example processes described. However, the present invention is not limited to the respective processes.

### AAV Downstream Processing workflow using functionalized filter material (addition of free functionalized filter material)

Figure 3 depicts an Example of a AAV purification process in downstream processing. Thereby, the following steps can be performed:
1. **Chemical Lysis & DNA Digestion:** The first step involves chemical lysis to break open cells and release AAVs, combined with DNA digestion to reduce nucleic acid contaminants. Here, enzyme-functionalized filter materials are used during lysis. The immobilized enzyme is more stable compared to free enzyme. Moreover, the handling during manufacturing can be simplified since only one addition of functionalized filter materials is needed compared to two distinct addition steps when filter materials and free enzyme are used instead.
2. **Depth Filtration/Clarification:** Following lysis & DNA digestion, depth filtration is performed, possibly with fewer number of depth filters needed due to the functionalized filter materials used in the previous step. Here, a 0.2 µm normal flow filtration (NFF) may be performed to clarify the harvest.
3. **Tangential Flow Filtration (TFF):** This step concentrates the AAV particles and exchanges the buffer, preparing it for further purification.
4. **Acidification & Bioburden Filtration:** Acidification is a prerequisite to purify AAV via cation-exchange chromatography. Capsids are acidified and their positive charge enables the purification via cation exchange. In addition, viral contaminants maybe inactivated and other contaminants like e.g. HCPs often precipitate in this step, which may be removed by 0.45 µm NFF.
5. **Capture (Cation-exchange Chromatography):** The AAV particles may be captured using cation-exchange chromatography. Due to previous steps, this may require fewer chromatography steps.
6. **Polishing (Anion-exchange Chromatography):** Further purification may be achieved through anion-exchange chromatography, which is aimed to achieve full particle enrichment. This step is more effective due to the previous optimization enabled by immobilized enzymes on the functionalized filter materials, which do not have to be purified from the process liquid.
7. **Concentration & Buffer Exchange (TFF):** The AAV solution may be further concentrated and may undergo another buffer exchange via TFF. Some stabilizers such as polysorbates may be added after TFF to achieve final formulation buffer.
8. **Sterile Filtration (0.2 µm NFF):** The final DSP step may be sterile filtration through a 0.2 µm NFF to ensure sterility of the AAV product.

### (Semi) Continuous Perfusion Processes (e.g. mAb, AAV, LV) with functionalized filter materials retained in incubation chamber

Figure 4 depicts a continuous perfusion process for the production of dedicated molecules (e.g. mAbs, or AAVs). Here all unit operations are performed in a (semi) continuous manner. The continuous digestion of host cell DNA is achieved by nuclease functionalized filter material in an incubation chamber. Functionalized filter materials are retained in the incubation chamber and can be used during the whole process duration.
1. **Cell Clarification:** The cell culture coming from the bioreactor is continuously clarified. This might for example be done using alternating tangential flow (ATF), tangential flow filtration (TFF), or tangential flow depth filtration (TFDF). The biopharmaceutical product (e.g. mAb, AAV, LV) remains in the clarified solution and is not retained in the clarification step.
2. **DNA Digestion:** The clarified solution is then loaded into an incubation chamber with nuclease-functionalized filter materials to digest DNA. Once the incubation chamber is loaded, the enzymatic digestion is performed for a fixed time frame. During the digestion process, the subsequent process liquid is held back in a holding tank or by pausing the ATF process. Once the digestion process is completed, the digested process liquid is discharged from the incubation chamber, while the functionalized filter materials are retained in the incubation chamber (e.g. by a membrane), which allows the reuse thereof. After the incubation chamber is emptied, it can be reloaded with process liquid from the holding tank or by restarting the ATF process. Once the target liquid level is achieved, another digestion cycle can be started. The retention of the functionalized filter material results in lower manufacturing costs, since the immobilized enzyme can be reused for several cycles.
3. **Tangential Flow Filtration (TFF):** An optional concentration step using TFF may be implemented to concentrate the target product if needed.
4. **Capture:** The target product is then captured for example by affinity chromatography or ion exchange (IEX) chromatography. Since nucleases are immobilized on the functionalized filter materials and retained in step 2, the nucleases don't need to be removed in this chromatography step, which simplifies this process step.
5. **Polishing:** Further purification may be achieved through anion-exchange chromatography, which is aimed to achieve full particle enrichment. Since nucleases are immobilized on the functionalized filter materials and retained in step 2, the nucleases don't need to be removed in this chromatography step, which simplifies this process step.
6. **Concentration & Buffer Exchange (TFF):** After polishing, there may be another TFF step required to concentrate the biomolecules and exchange the buffer with the final formulation buffer. Some stabilizers such as polysorbates may be added after TFF.
7. **Sterile Filtration (0.2 µm NFF):** Lastly, the product may be filtered through a 0.2 µm NFF to ensure sterility before it can be released for clinical use.

### (Semi) Continuous Perfusion Processes (e.g. mAb, AAV) with functionalized filter materials incorporated in ATF/TFF/TFDF Cassettes

Figure 5 depicts a continuous perfusion process for the production of dedicated molecules (e.g. mAbs, or AAVs). Here all unit operations are performed in a continuous manner. The continuous digestion of host cell DNA is achieved by nuclease functionalized filter materials, which are incorporated in a ATF, TFF or TFDF module. Thus, nucleases are retained and can be used throughout the process. An additional incubation step with nucleases might be skipped.
1. **Cell Clarification:** The cell culture provided from the bioreactor is continuously clarified. This can be achieved by using alternating tangential flow (ATF), tangential flow filtration (TFF), or tangential flow depth filtration (TFDF). ATF, TFF or TFDF is combined with a layer incorporating functionalized filter materials, which digests the host cell DNA continuously. Due to low process flow rates in perfusion processes, the residence time of cell culture supernatant in these modules is sufficient to digest the DNA. The biopharmaceutical product (e.g. mAb, AAV, LV) remains in the clarified solution and is not retained in the clarification step.
2. **Tangential Flow Filtration (TFF):** An optional concentration step using TFF may be implemented to concentrate the target product if needed.
3. **Capture:** The target product is then captured for example by affinity chromatography or ion exchange (IEX) chromatography. Since nucleases are immobilized on the filter materials and retained in step 1, the nucleases don't need to be removed in this chromatography step, which simplifies this process step.
4. **Polishing:** Further purification may be achieved through anion-exchange chromatography, which is aimed to achieve full particle enrichment. Since nucleases are immobilized on the FA and retained in step 2, the nucleases don't need to be removed in this chromatography step, which simplifies this process step.
5. **Concentration & Buffer Exchange (TFF):** After polishing, there may be another TFF step required to concentrate the biomolecules and exchange the buffer to the final formulation buffer. Some stabilizers such as polysorbates may be added after TFF.
6. **Sterile Filtration (0.2 µm NFF):** Lastly, the product may be filtered through a 0.2 µm NFF to ensure sterility before it can be released for clinical use.

### E. coli clarification for plasmid purification applying functionalized filter materials

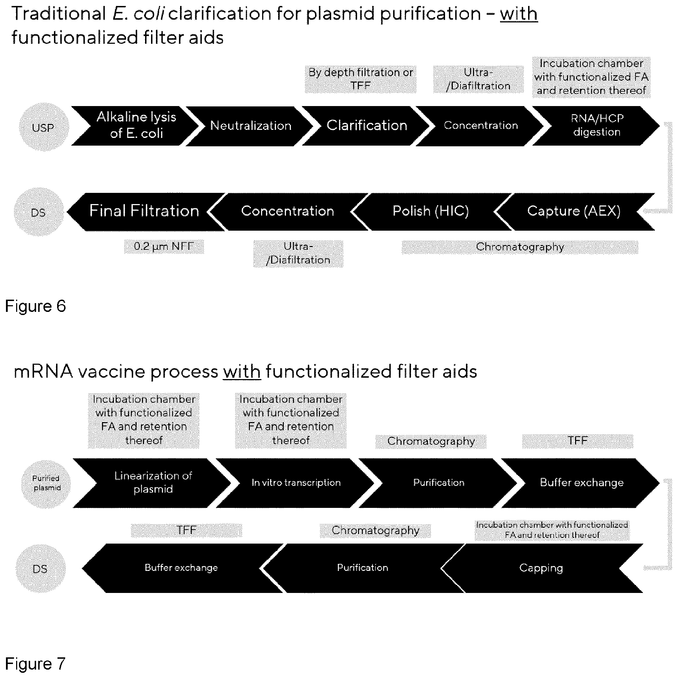

Figure 6 depicts a process starting with a culture of *E*. *coli* that has been genetically engineered to produce a desired plasmid DNA (pDNA). Thereby, the following steps can be performed:
1. **Alkaline Lysis of *E. coli:*** The *E*. *coli* cells undergo alkaline lysis, where an alkaline solution lyses the cells to release their contents, including the pDNA.
2. **Neutralization:** The lysate may then be neutralized, which causes cell debris and genomic DNA to precipitate out of solution, leaving plasmid DNA in the supernatant.
3. **Clarification:** The mixture is clarified to remove the precipitated cell debris and genomic DNA. This can be achieved by depth filters, TFF processes, centrifugation, or combinations thereof.
4. **Ultra-/Diafiltration:** The plasmid DNA may be subjected to ultrafiltration for concentration and/or diafiltration to remove small molecules and exchange the buffer, further purifying the plasmid DNA.
5. **RNA/HCP digestion:** The concentrated plasmid DNA is transferred into an incubation chamber with nuclease-functionalized and/or protease-functionalized filter materials to digest RNA or HCPs. Once the incubation chamber is fully loaded, the enzymatic digestion is performed for a fixed time frame. During the digestion process, the subsequent process liquid is held back in a holding tank. Once the digestion process is completed, the digested process liquid is discharged from the incubation chamber, while the functionalized filter materials are retained in the incubation chamber (e.g. by a membrane), which allows to reuse the functionalized filter material. The incubation chamber is loaded with stored process liquid from the holding tank until it is filled to initiate another digestion cycle. The use of functionalized RNAase filter materials is especially useful, if produced plasmid DNA is intended to be used for further *in vitro* transcription processes (mRNA), since the risk to carry over RNAase into the final plasmid DNA product would be dramatically decreased.
6. **Capture (Anion Exchange Chromatography** - **AEX):** The plasmid DNA is then captured using anion exchange chromatography, which exploits the negative charge of DNA to bind it to positively charged groups on a chromatographic resin.
7. **Polish (Hydrophobic Interaction Chromatography** - **HIC):** After capture, plasmid DNA may be polished to remove any remaining impurities using hydrophobic interaction chromatography, which separates molecules based on their hydrophobicity.
8. **Concentration & Buffer Exchange (TFF):** After polishing, there may be another TFF step required to concentrate the biomolecules and exchange the buffer to prepare the final formulation. Some stabilizers such as polysorbates may be added after TFF.
9. **Final Filtration (0.2 µm NFF):** The purified plasmid DNA may undergo a final sterile filtration step through a 0.2 µm normal flow filter to ensure the removal of any remaining contaminants, leading to the final drug substance (DS).

### mRNA vaccine process with functionalized filter materials

Figure 7 depicts a process starting with a purified plasmid DNA (pDNA), which contains the genetic code for the desired mRNA sequence of the target antigen or protein. Thereby, the following steps can be performed:
1. **Linearization of pDNA:** In an incubation chamber with enzyme-functionalized filter materials, the pDNA undergoes an enzymatic reaction to linearize it, which is a preparation step for transcription.
2. **In Vitro Transcription:** The linearized pDNA is transcribed in vitro to synthesize the mRNA strand, again utilizing functionalized filter materials to stabilize the reaction and retain the necessary enzymes within the chamber.
3. **Purification by chromatography:** After transcription, the mRNA is subjected to a purification step by e.g. anion-chromatography to remove unincorporated nucleotides, enzymes, and other residuals from the transcription process.
4. **Tangential Flow Filtration (TFF) for Buffer Exchange:** Following chromatography, TFF may be used to exchange the buffer, optimizing the conditions for the final steps of the vaccine preparation.
5. **Capping:** The mRNA undergoes another enzymatic reaction in an incubation chamber with functionalized filter materials, where a cap structure is added to the mRNA. This cap is crucial for the mRNA's stability and efficient translation in the host cells.
6. **Purification by chromatography:** This step might be omitted when employing functionalized filter materials for capping, since capping-enzyme is retained on functionalized filter materials. However, if leachable of capping enzyme is too high another chromatography step such as anion-chromatography might be required.
7. **Concentration & Buffer Exchange (TFF):** After capping/purification, another TFF step may be required to concentrate the biomolecules and exchange the buffer to formulate mRNA.
8. **Final Drug Substance (DS):** The capped mRNA is the final drug substance, ready for formulation into the vaccine delivery system.

### AAV Downstream Processing workflow using functionalized filter material (use of functionalized filter material embedded in a depth filter)

Figure 8 depicts an Example of a AAV purification process in downstream processing. Thereby, the following steps can be performed:
1. **Chemical Lysis & DNA Digestion:** The first step involves chemical lysis to break open cells and release AAVs
2. **Depth Filtration/Clarification:** Following lysis, depth filtration with embedded functionalized filter materials is performed. Fewer number of depth filters may be required due to the functionalized filter materials embedded into the depth filters. Host cell DNA is digested in this process step by the functionalized filter material embedded in the depth filter. Subsequently, a 0.2 µm normal flow filtration (NFF) may be performed to further clarify the harvest.
3. **Tangential Flow Filtration (TFF):** This step concentrates the AAV particles and exchanges the buffer, preparing it for further purification.
4. **Acidification & Bioburden Filtration:** Acidification is a prerequisite to purify AAV via cation-exchange chromatography. Capsids are acidified and their positive charge enables purification via cation exchange. In addition, viral contaminants may be inactivated and other contaminants like e.g. HCPs often precipitate in this step, which may be removed by 0.45 µm NFF.
5. **Capture (Cation-exchange Chromatography):** The AAV particles may be captured using cation-exchange chromatography. Due to previous steps, this may require fewer chromatography steps.
6. **Polishing (Anion-exchange Chromatography):** Further purification may be achieved through anion-exchange chromatography, which is aimed to achieve full particle enrichment. This step is more effective due to the previous optimization enabled by immobilized enzymes on the functionalized filter materials, which do not have to be purified from the process liquid.
7. **Concentration & Buffer Exchange (TFF):** After polishing, there may be another TFF step required to concentrate the biomolecules and exchange the buffer to the final formulation buffer. Some stabilizers such as polysorbates may be added after TFF.
8. **Sterile Filtration (0.2 µm NFF):** The final DSP step may be sterile filtration through a 0.2 µm NFF to ensure sterility of the AAV product.

### Continuous Perfusion Processes (e.g. mAb, AAV, LV) with functionalized filter materials retained in serially connected incubation chambers

Figure 11 depicts a continuous perfusion process for the production of dedicated molecules (e.g. mAbs, or AAVs). Here, all unit operations are performed in a continuous manner. The continuous digestion of host cell DNA is achieved by nuclease functionalized filter material in multiple incubation chambers, which are connected in series. Functionalized filter materials are retained in the incubation chambers and can be used during the whole process duration.
1. **Cell Clarification:** The cell culture provided from the bioreactor is continuously clarified. This can for example be achieved using alternating tangential flow (ATF), tangential flow filtration (TFF), or tangential flow depth filtration (TFDF). The biopharmaceutical product (e.g. mAb, AAV, LV) remains in the clarified solution and is not retained in the clarification step.
2. **DNA Digestion:** The clarified solution is then loaded into an incubation chamber with nuclease-functionalized filter materials to digest DNA. Once the target level of liquid is achieved, the enzymatic digestion takes place for a fixed time frame. During the digestion period in incubation chamber 1, the subsequent process liquid is directed into a second incubation chamber using a switching valve. The second incubation chamber is loaded, while the digestion takes place in incubation chamber 1. After the digestion in incubation chamber 1 is completed, the digested process liquid is discharged from the incubation chamber into the next process unit. Functionalized filter materials retained in the incubation chamber (e.g. by a membrane) are used for the subsequent process liquid, which will be directed to the emptied incubation chamber once the other incubation chamber is full. This allows to perform the digestion for a fixed minimum time period, while a continuous process liquid flow can be enabled. Since the functionalized filter material can be reused, the manufacturing costs can be reduced as less nuclease is needed.
3. **Tangential Flow Filtration (TFF):** An optional concentration step using TFF may be implemented to concentrate the target product if needed.
4. **Capture:** The target product is then captured for example by affinity chromatography or ion exchange (IEX) chromatography. Since nucleases are immobilized on the functionalized filter materials and retained in step 2, the nucleases don't need to be removed in this chromatography step, which simplifies this process step.
5. **Polishing:** Further purification may be achieved through anion-exchange chromatography, which is aimed to achieve full particle enrichment. Since nucleases are immobilized on the functionalized filter materials and retained in step 2, the nucleases don't need to be removed in this chromatography step, which simplifies this process step.
6. **Concentration & Buffer Exchange (TFF):** After polishing, there may be another TFF step required to concentrate the biomolecules and exchange the buffer to the final formulation buffer. Some stabilizers such as polysorbates may be added after TFF.
7. **Sterile Filtration (0.2 µm NFF):** Lastly, the product may be filtered through a 0.2 µm NFF to ensure sterility before it can be released for clinical use.

## Claims

1. A biopharmaceutical production process comprising a step of contacting a process liquid with a functionalized filter material, wherein the functionalized filter material comprises a filter material and one or more hydrolases covalently bonded to the filter material.

2. The biopharmaceutical production process according to claim 1, wherein the hydrolase of the functionalized filter material is selected from the group consisting of an esterase, such as a carboxylesterase and a cholinesterase, a lipase, a phospholipase, a peptidase, such as a serine protease, a cysteine protease, an aspartic protease, and a metalloprotease, a nuclease, such as a DNase and a RNase, a glycoside hydrolase, such as an amylase, a cellulase, a lactase, and a pectinase, a phosphatase, such as an alkaline phosphatase and an acid phosphatase, a sulfatase, such as an arylsulfatase, an amidase, such as an urease and an asparaginase, and an epoxide hydrolase, such as a microsomal epoxide hydrolase, as well as combinations of two or more thereof.

3. The biopharmaceutical production process according to claim 2, wherein the hydrolase of the functionalized filter material is a nuclease.

4. The biopharmaceutical production process according to any one of claims 1 to 3, wherein the filter material of the functionalized filter material is selected from the group consisting of diatomaceous earth, perlite, cellulose fibers, activated carbon, silica gel, glass microfibers, polymeric resins, minerals, such as clays, pumice, and zeolites, preferably diatomaceous earth, perlite, cellulose fibers, and zeolites.

5. The biopharmaceutical production process according to claim 4, wherein the filter material of the functionalized filter material is diatomaceous earth.

6. The biopharmaceutical production process according to any one of claims 1 to 5, wherein the filter material of the functionalized filter material has a permeability of from 20 mD to 5000 mD.

7. The biopharmaceutical production process according to any one of claims 1 to 6, wherein the functionalized filter material performs adsorptive and enzymatic degradation of components other than a biopharmaceutical product of interest in the process liquid.

8. The biopharmaceutical production process according to any one of claims 1 to 7, wherein the contacting step is carried out during or directly before or after a clarification step.

9. The biopharmaceutical production process according to claim 8, wherein, in the clarification step, the process liquid is subjected to one or more of centrifugation, depth filter filtration, normal flow filter filtration, alternating tangential flow (ATF) filtration, tangential flow filtration (TFF), and tangential flow depth filtration (TFDF).

10. The biopharmaceutical production process according to any one of claims 1 to 9, wherein the contacting step is carried out before or after a clarification step, and wherein the functionalized filter material is included in an incubation chamber.

11. The biopharmaceutical production process according to claim 8 or 9, wherein the contacting step is carried out during a clarification step, wherein the functionalized filter material is embedded in a filter medium.

12. The biopharmaceutical production process according to claim 11, wherein the filter medium is selected from single- or multi-layered fibers selected from the group consisting of glass microfibers, cellulose fibers, polyamide fibers, polyethersulfon (PES) fibers, polytetrafluoroethylene (PTFE) fibers, polyvinylidenefluoride (PVDF) fibers, polypropylene (PP) fibers, and polysulfone (PSU) fibers.

13. The biopharmaceutical production process according to any one of claims 1 to 12, wherein the contacting step is carried out before one or more chromatography steps selected from affinity chromatography, ion exchange (IEX) chromatography, mixed mode chromatography, and reverse phase chromatography.

14. The biopharmaceutical production process according to any one of claims 1 to 13, wherein a biopharmaceutical product obtained by the biopharmaceutical production process is selected from therapeutic proteins, such as mAbs, viral vectors, such as AAV, viruses, such as lentivirus, nucleic acid based products, such as mRNA, pDNA, olignucleotides, and siRNA, exosomes, such as extracellular vesicles, and lipid-nano-particles, such as RNA vaccines and liposomes.

15. The biopharmaceutical production process according to any one of claims 1 to 14, further comprising, in addition to the first contacting step, one or more additional contacting step(s) before or after the first contacting step, wherein the functionalized filter material(s) applied in the additional contacting step(s) are independently the same or different than the functionalized filter material of the first contacting step.
